# EUROPEAN PATENT SPECIFICATION

(11) **EP 4 483 916 B1**
(45) Date of publication and mention of the grant of the patent: **25.11.2026**
(21) Application number: 23181634.9
(22) Date of filing: 27.06.2023
(51) Int. Cl.: A61M 1/16, A61M 1/36, A61M 60/109, A61M 60/113, A61M 60/232, A61M 60/279, A61M 60/38, A61M 60/569

(54) **COMBINED BLOOD PUMP AND OXYGENATOR SYSTEM**
KOMBINATION AUS BLUTPUMPE UND OXYGENATORSYSTEM
SYSTÈME COMBINÉ DE POMPE À SANG ET D'OXYGÉNATEUR

(43) Date of publication of application: 01.01.2025
(73) Proprietor: BIOxy Med LLC-FZ, Dubai (AE)
(72) Inventor: ABOULHOSN, Walid, 00000 Btekhnay (LB); KARAMEH, Fadi, 5447 AinZhalta (LB); RAFEH, Nidal Abi, Covington, Louisiana, 70433 (US); SALHA, Sary, 00000 Ras el Maten (LB); EL BASHA, Rawan, 5673 Dmit (LB); EL GHOSAYNI, Hadi, 5728 Baakleen (LB); SHKEIR, Faysal, 00000 Ras el Maten (LB); HADDAD, Jad, 00000 Ain dara (LB); HARB, Hasan, 00000 Gharifeh (LB); MAHMOUD, Yasser, 00000 Srayra (LB)
(74) Representative: Platzöder Patentanwaltsgesellschaft mbH

(56) References cited:
- EP-B1- 2 679 257
- EP-B1- 3 624 869
- WO-A2-2022/187625
- GB-A- 2 589 689
- US-A1- 2017 252 505
- US-A1- 2020 000 999
- US-B2- 10 188 780
- US-B2- 9 011 311

## Description

### BACKGROUND OF THE INVENTION

### Technical Field

The present invention relates generally to the field of blood pumps and oxygenators used to increase perfusion and oxygen level in a patient. More specifically, the present invention can provide a pump-oxygenator system that is easy to set up and use, is compact in size and may allow for increased mobility of a patient.

### Background Art

Various types of blood pump-oxygenator systems are well known in the art. For example, during open-heart surgery, the patient is interconnected with an external pump-oxygenator system, commonly known as a heart-lung machine, which circulates blood and introduces oxygen into the blood system. Most types of blood pumps employ a roller or a centrifugal pump to convey blood in the patient's vascular system and/or through an extracorporeal system. Most types of oxygenators use a gas-permeable membrane, wherein blood flows along one side of the membrane and oxygen is supplied to the other side of the membrane. Given a sufficient pressure gradient between the oxygen supply and the blood, the oxygen will diffuse through the membrane and into the blood. In addition, carbon dioxide will tend to diffuse from the blood into the membrane. The membrane may be provided in the form of hollow fibers.

Such systems may be referred to as extracorporeal membrane oxygenation (ECMO), also known as extracorporeal life support (ECLS). This is an extracorporeal technique of providing prolonged cardiac and respiratory support to persons whose heart and lungs are unable to provide an adequate amount of gas exchange or perfusion to sustain life. The technology for ECMO is largely derived from cardiopulmonary bypass, which provides shorter-term support with arrested native circulation. Typical cardiopulmonary bypass systems are rather complex, and generally are not particularly well adapted for applications longer than six hours. Moreover, most standard systems exhibit poor hemodynamic characteristics. That is, such systems typically cause too much damage to the blood to be useful for extended periods.

Cardiopulmonary bypass systems are disclosed for example in US 3,890,969, US 4,466,804, and US 4,610,656. Such conventional systems commonly utilize several pumps, a venous reservoir, an arterial reservoir, and a separate bubble-trapping device. These conventional systems exhibit several disadvantages. The most apparent disadvantage is the overall complexity of such systems. For example, the numerous components require extensive tubing and interconnections.

The complexity of the conventional systems leads to higher costs of manufacture and operation. Also, complex systems may take longer to set up, and system set-up may require expert personnel and supervision. Even with such expert personnel present, a system's complexity increases the risk of error in setting up the system. Likewise, once in operation, a conventional system requires continuous monitoring and adjustment by expert personnel.

Conventional cardiopulmonary bypass systems are also not usable for long-term application because they significantly damage the blood after a fairly short use (e.g., 6-8 hours). For instance, conventional occlusive roller pumps mechanically destroy red blood cells. This "blood trauma" can occur in any cardiopulmonary bypass system. It is caused and/or aggravated by occlusive pumps, interconnections, and other system components likely to increase system pressure or turbulence. Similarly, using conventional oxygenators for beyond a few hours to a day results in diminished performance due to plasma leakage into the hollow fibers.

The above factors illustrate the need for and desirability of a simple cardiopulmonary bypass system with relatively few components. Devices similar to that disclosed in US 4,540,399 represent attempts to achieve simplicity. There it is disclosed an emergency bypass system with one non-occlusive pump, an oxygenator, and a separate bubble trap. It is described a centrifugal rotor-type pump connected proximal to (i.e., on the venous side of) the oxygenator. While this may represent an attempt to provide a simplified cardiopulmonary bypass system compared to other known systems, it still may be considered to need further improvement.

In other situations, a smaller, implantable oxygenator may be sufficient to adequately supplement the patient's cardiopulmonary function by marginally increasing the oxygen content of the patient's blood. For example, patients suffering from emphysema, pneumonia, congestive heart failure, or other chronic lung disease often have blood oxygen partial pressures of approximately 40 torr. A relatively small increase of 10% to 20% is generally sufficient to adequately maintain the patient. This is a particularly desirable alternative in that it can avoid the need to intubate the patient. In addition, temporary use of this type of oxygenator may be sufficient in many cases to tide the patient over an acute respiratory insult. Placing such patients on a conventional respirator is often the beginning of a progressive downhill spiral by damaging the patient's pulmonary tree, thereby causing greater dependence on the respirator.

A number of devices and processes have been invented in the past incorporating this basic technology, including the following. US 3,505,686 demonstrates the general concept of using gas permeable fibers to boost the oxygen level of blood. In the implantable embodiment of this disclosure, a tubular casing serves as a shunt either from the pulmonary artery to the left atrium of the heart, or more generally between an artery and a vein. A multitude of parallel-connected capillary tubes are used to oxygenate and/or purify the blood circulating through the casing. US 4,583,969 shows a transvenous oxygenator made of a plurality of small diameter gas permeable tubes. However, in this specific device disclosed two incisions may be required. The insertion process is also rather complex. US 4,631,053 discloses a transvenous oxygenator having a single membrane through which oxygen diffuses. The membrane is disposed within a sheath and a flexible wire supports both.

WO 2021/170712 A1 discloses a combined blood pump and oxygenator system and related methods. A blood pump-oxygenator system is provided that comprises at least one blood pump, an oxygenator, an inflow cannula, and an outflow cannula, which are connected so as to form a closed series circuit to be operable as a cardiopulmonary bypass system adapted for extracorporeal processing of the patient's blood. The blood pump is configured to convey blood through the circuit from the patient into the inflow cannula, through the oxygenator and out of the outflow cannula back into the patient. A manifold is connected between the inflow cannula and the outflow cannula so that the blood passes through the manifold, wherein the manifold accommodates the blood pump and the oxygenator and is designed so as to form a recirculation loop configured to recirculate at least part of the blood in the circuit such that the blood passes over the oxygenator multiple times. The system further comprises an extra blood pump positioned at the outflow cannula and configured to deliver a set volume to the patient, wherein the extra pump is configured to be controlled independently from the blood pump that circulates the blood in the manifold including the oxygenator.

Recently, a number of centers are implementing ambulatory ECMO support using available pumps and oxygenators, allowing patients to walk, eat and exercise. This is an improvement because it allows patients to move, and participate in physical rehabilitation, preventing muscles from atrophy and ultimately leading to a better clinical course to recover or have a better status at the time of heart/lung transplantation.

Currently available devices may be very bulky and multiple exchanges of oxygenators are required for long-term support, which usually cannot be done without the interruption of the whole system. Moreover, the rigid nature of the currently available oxygenators usually defines the circulation track of the blood, which restricts the circulation of blood to be divided all over the area of oxygenator. This causes fast decay of the same hollow fibers where the blood is always circulating, and decreases the lifetime of the system as a whole.

Furthermore, known ECMO systems typically comprise a single oxygenator, wherein, e.g., 100% pure oxygen or a gas mixture of, e.g., 80% oxygen and 20% air is supplied at any time through the hollow fibers in order to both, increase the oxygen level in the blood and remove carbon dioxide from the blood and from the oxygenator. However, this requires large flow rates of the gas mixture, e.g., up to 15 L / min. This in turn requires a large amount of pure oxygen, which is typically delivered from bulky and heavy oxygen gas bottles restricting the patient's mobility.

A need exists for a circulatory support system capable of supporting a patient in circulatory dysfunction for some length of time. Such a circulatory support system could be quite useful in numerous situations, such as (1) where the patient is in a state of cardiogenic shock; (2) where the patient is in a state of septic shock, (3) for post cardiotomy weaning from the bypass, (4) for assisting the circulatory system to avoid an impending myocardial infarction; and (5) bridge-to-transplant patients. It may further be desirable to provide a circulatory support system that can be used in an ambulatory environment and provides mobility for the patient.

### SUMMARY OF THE INVENTION

It is an object of the present invention to provide an improved blood pump and oxygenator system. Specifically, the present invention is directed at addressing at least one of the above-mentioned needs and eliminating, or at least reducing, at least one of the shortcomings of the prior art systems as described above.

A solution to this problem is provided by the teaching of the independent claims. Various preferred embodiments of the present invention are provided by the teachings of the dependent claims.

According to an aspect, a blood pump-oxygenator system for increasing perfusion and oxygen level in a patient comprises a blood flow inlet and a blood flow outlet which are connected so as to form a circuit to be operable as a cardiopulmonary bypass system adapted for extracorporeal processing of the patient's blood, a first blood pump and a first oxygenator, wherein the first blood pump is configured to convey blood through the circuit from the patient into the blood flow inlet, through the first oxygenator and out of the blood flow outlet back into the patient. The system further comprises a second blood pump and a second oxygenator, wherein the second blood pump is configured to convey blood through the circuit from the patient into the blood flow inlet, through the second oxygenator and out of the blood flow outlet back into the patient. The circuit comprises a first branch and a second branch arranged in parallel with each other, the first branch accommodating the first blood pump and the first oxygenator, and the second branch accommodating the second blood pump and the second oxygenator. The system also comprises a pump control unit, wherein the first and second blood pumps are configured to be controlled by the pump control unit to increase or decrease a flow rate of blood through the respective oxygenator in such a manner to vary a ratio of flow rates of blood through the first and second oxygenators.

In the system of the present invention, two blood pumps and two oxygenators are provided which are arranged in parallel branches, i.e. the pumps and oxygenators can be operated in parallel. More specifically, a ratio of the flow rates of blood through the oxygenators can be changed, which allows to efficiently use both oxygenators. In this way, the oxygenation of the blood conveyed through the system, in particular through the oxygenators can be improved as the dwell time of the blood in the oxygenators can be controlled. This in turn allows for a compact design of the system thereby increasing a mobility level of the patient. More specifically, the dwell time, i.e., the period of time during which the blood is inside the oxygenator, can be increased as will be described in more detail below. For instance, the dwell time can be controlled by slowing down the blood flow through one oxygenator and speeding the blood flow through the other oxygenator.

The system of the present invention can provide partial veno-arterial cardiopulmonary support over a relatively long period (i.e., one to ten days) because of its very low overall blood trauma and improved ease of use characteristics. The system is capable not only of providing cardiopulmonary support but also of providing hemodynamic support over a potentially long period. The system of the present invention is particularly adapted for partial hemodynamic support. Notwithstanding this, it is to be readily appreciated that the system is also suitable for achieving a number of additional objectives in a variety of situations involving circulatory dysfunction. The system of the present invention can assist circulation during relatively mild circulatory dysfunction, and also can provide acute assistance during severe refractory cardiac failure caused by myocardial infarction, cardiomyopathy, or post-cardiac surgery. The system of the present invention may also be useful in providing perioperative support and stabilization of high-risk patients, such as coronary bypass surgery patients.

The system of the present invention may also be useful in causing less damage to the blood than conventional systems by reducing the surface area the blood interacts with, making the system of the present invention more suitable for long-term applications. Trauma to the patient's blood can be reduced principally by the simplicity of a blood circuit, especially a low number of circuit elements, a non-occlusive pump and a design of the oxygenator(s). In addition, overall trauma to the patient can be reduced. This can be accomplished by reducing the extracorporeal processing of the blood as compared to conventional bypass systems and procedures. The absence of separate fluid reservoirs can reduce the fluid volume of the system. Because less of the patient's blood is withdrawn from the body at one time, the overall trauma to the patient can be reduced.

The low trauma of the system contributes desirably to overall hemodynamic characteristics. Many of the features of the present invention that improve the overall hemodynamics of the system may also help to simplify the system, and make it more reliable and compact. To further reduce the risk of system malfunction, the system may be equipped with various fail-safe features. Such equipment may be used to monitor and control various pressures and flow rates to ensure proper operation. Although the system of the present invention may require periodic monitoring and adjustment by expert personnel during operation, it can be operated relatively automatically as will be described in more detail below. That is, compared to conventional systems, which tend to be more complex, the system of the present invention requires much less monitoring and adjustment during operation.

The term "oxygenator", as used herein, particularly refers to a medical device that is configured to oxygenate blood and remove carbon dioxide from the blood. It is used to maintain gas exchange, e.g., in cardiac surgery as part of a heart-lung machine or in critical care medicine to treat acute respiratory failure. The oxygenator briefly replaces the function of the lungs. The oxygenator may be specifically a membrane oxygenator. Such oxygenator may have hollow fibers, such as hair-like fibers. The fibers may be arranged in "sets" or "bundles", wherein fibers of one set may have a common inlet and outlet respectively. The oxygenator may comprise a plurality of hollow oxygenation fibers that allow for blood to pass between the oxygenator fibers to intake oxygen and release carbon dioxide, while oxygen and/or air is passed through an inside lumen of the oxygenator fibers from the inflow lumen at one end of the oxygenator fibers and returned from the other end of oxygenator fibers to the outflow lumen. Oxygen and air may be delivered to the oxygenator via an inflow tube connected to the inflow, and an outflow tube connected to the outflow may be provided for removal of carbon dioxide from the oxygenator.

The term "blood pump" (or simply "pump"), as used herein, particularly refers to a medical device for pumping blood, specifically through an extracorporeal system. The blood pump may be a rotary pump or a displacement pump, such as a rotary pump of the centrifugal type or the axial type, wherein the blood pump may be configured to be directly driven by an electric motor or to be driven by a flexible drive cable that links the blood pump to the electric motor either magnetically or directly. A rotor may be situated inside a pump housing, wherein a drive cable may be inside a sheath coupled to the rotor in such a manner that rotation of the drive cable by an electric motor causes rotation of the rotor and pumping of blood from the distal end to the proximal end of housing so as to transport the blood from the blood flow inlet toward the blood flow outlet.

If applicable, the terms "first", "second", "third" and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Where the term "comprising" or "including" is used in the present description and claims, it does not exclude other elements or steps. Where an indefinite or definite article is used when referring to a singular noun e.g., "a" or "an", "the", this includes a plural of that noun unless something else is specifically stated.

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In the following, further preferred embodiments of the system are described, which can be arbitrarily combined with each other or with other aspects of the present disclosure, unless such combination is explicitly excluded or technically impossible.

In some embodiments, the first and second blood pumps are configured to be controlled by the pump control unit to increase or decrease the flow rate of blood through the respective oxygenator in a synchronized and opposite manner. In some embodiments, the first and second blood pumps are configured to be controlled by the pump control unit to increase or decrease the flow rate of blood through the respective oxygenator while a total flow rate remains at a required level. In this manner, the overall amount of blood that is conveyed through the system does not necessarily change but the dwell time in the respective oxygenators can be increased. More specifically, while the blood flow is increased in one oxygenator, the blood flow in the other oxygenator is decreased, and vice versa. This allows to enhance the oxygenation for a longer period of time in one oxygenator while the other one is emptied, thereby ensuring that the blood flow to the patient is not affected.

In some embodiments, the system further comprises a gas control unit, wherein the gas control unit is configured to control a flow of oxygen as well as a flow of air to the first and second oxygenators in such a manner to vary a ratio of flow rates of oxygen and air through the first and second oxygenators, respectively. For instance, the gas control unit may control a four-way mixing valve. Controlling the flow rates of gas may contribute that the amount of need (pure) oxygen (O₂ gas) can be significantly reduced, in particularly compared to common systems having only one oxygenator. In such common systems a high flow rate of, e.g., 15 L/min of an oxygen-air mix (such as 80% oxygen and 20% air) may be needed at all times to ensure on the one hand that a sufficient amount of oxygen is received by the blood and on the other hand that carbon dioxide (CO₂) can be sufficiently removed and the oxygenator is purged from carbon dioxide. The gas control unit allows for switching between oxygen and air, thereby being able to reduce the total amount of need oxygen. For instance, at one time a high ratio of air can be supplied to wash out the oxygenator, while at another time high rate of oxygen can be supplied to achieve a sufficient oxygenation of the blood. This also allows for repeated washing of the oxygenators, preferably using only air. Furthermore, there are no dedicated fibers in the oxygenators that are used either only for oxygen or only for air, such that the entire bundle of fibers can be efficiently used thereby reducing non-uniform degradation of the oxygenator. The gas control unit may be configured to be synchronized with the pump control unit. For instance, a maximum amount of oxygen may be supplied to the one of the oxygenators in which the blood flow is slowed down, while the other oxygenator is emptied from blood and washed out by supplying a maximum amount of air.

In some embodiments, the system further comprises an oxygen concentrator for the supply of oxygen to the first and second oxygenators and an air compressor for the supply of air to the first and second oxygenators. This may eliminate the need for bulky oxygen bottle. Air may be simply taken from ambient air using a simple pump.

In some embodiments, the system further comprises a valve in each of the branches between the respective blood pump and the respective oxygenator to prevent blood from flowing in a backward direction from the oxygenator to the blood pump. Particularly in an oxygenator in which the blood flow is slowed down, the pressure in the parallel branch where the blood flow is higher may push the blood in a backward direction. This can be prevented by providing respective valves. Such valves can be simple non-return valves (check valves).

In some embodiments, the circuit comprises a bifurcation that diverts the blood flow in the circuit into the two branches, and a junction where the at least two branches are brought together, wherein at least one of the bifurcations and the junction is configured as a multi-way control valve that is operable so as to select at least one of the two branches for the blood to flow through. The provision of multi-way control valves, such as three-way-valves or four-way-valves, allows to control the proportion of blood that flows through each branch, and thus through each of the oxygenators. In particular, the valves may be controlled such that blood flows only through one of the branches at the same time. If multi-way valves are provided at each of the bifurcation and the junction, i.e., at the beginnings and ends of the parallel branches, the valves may be synchronized to establish a flow path through only a single branch at the same time. The valves may also be synchronized with the pumps. Thus, the dwell time of the blood in one oxygenator can be increased, while the other oxygenator is emptied. Furthermore, an oxygenator can be replaced easily by blocking the respective branch, while the other branch, and thus the other oxygenator, can continue to operate. The possibility of swapping oxygenators without the need to stop the system increases the usefulness of the system for long-term applications. Oxygenators in general tend to degrade in function very rapidly over time. Therefore, the possibility to change oxygenators without interrupting patient support may increase the overall lifetime of the system.

A multi-way control valve may have multiple ports (openings), wherein one of the ports may act as an inlet or outlet whereas the remaining ports act respectively as outlets and inlets respectively. For instance, a three-way control valve has three ports, through whose openings volume flows enter or leave. They may be used as mixing valves or as diverting valves (also referred to as switching valves). The flow rate may be regulated by an electric, pneumatic or electro-pneumatic actuator. A three-way mixing valve has two inlets and one outlet, wherein two flows, particularly the flows from the branches at the junction, flow into the valve and leave it mixed via the third way. In a diverting valve (one inlet and two outlets), the incoming volume flow is distributed to two outlets, leading to the branches at the bifurcation.

In some embodiments a reservoir is provided at the junction, wherein the reservoir comprises at least two inlet ports, an outlet port and at least one vent. Each of the branches ends in the reservoir at a respective inlet port such that all blood from the branches and thus the oxygenators is fed to and mixed in the reservoir. The collected blood exits the reservoir at the outlet port, wherein the outlet port is extended towards an inside of the reservoir. The at least one vent is configured for exhausting gas bubbles in the blood in the reservoir. Thus, the reservoir may also be referred to as gas bubble removing device or "bubble trap". It is advantageous to remove bubbles at this point in the system by means of the reservoir because it can be avoided that gas bubbles are further circulated even if gas bubbles may be removed by a bubble trap at the blood flow outlet as described below.

Particularly, a basic principle for removing gas bubbles may be that the reservoir is in a position during operation where the vent(s) is/are in an upper portion thereof as the gas bubbles will rise upwards. The inlet port(s) is in an upper portion, too, while the outlet port is designed in such a way that it takes blood from a location that is below the vent, e.g., by means of a tube or the like that extends to the inside of the reservoir, e.g., towards the center or further to a lower portion of the inside of the device. Exemplary embodiments will be described in more detail below with respect to the drawings.

In some embodiments, the system further comprises a first oxygen sensor, a second oxygen sensor and a third oxygen sensor, all configured to measure an oxygen level in the blood. The first oxygen sensor is positioned upstream of the first and second oxygenators, particularly upstream of the bifurcation (or at or near the blood flow inlet). The second oxygen sensor is positioned in the first branch downstream of the first oxygenator so as to obtain an oxygenation rate of the first oxygenator by a comparison of an oxygen level measured by the first oxygen sensor with an oxygen level measured by the second oxygen sensor. The third oxygen sensor is positioned in the second branch downstream of the second oxygenator so as to obtain an oxygenation rate of the second oxygenator by a comparison of an oxygen level measured by the first oxygen sensor with an oxygen level measured by the third oxygen sensor. This allows to monitor the function of the oxygenators. It will be appreciated that it may be preferable to provide an oxygen sensor downstream of each oxygenator in each branch to be able to monitor the oxygenation level of the blood after it has passed the respective oxygenator, which may be used to monitor degradation of the oxygenators. However, a single oxygen sensor upstream of the oxygenators (downstream of the junction, or at or near the blood flow outlet) may be sufficient. In this case only a total oxygenation level of both oxygenators can be monitored.

In some embodiments, the first and second blood pump may be configured to be controlled such that a pump rate of the blood pumps is set depending on the respectively obtained oxygenation rate. For instance, a pump rate can be increased when the oxygenation rate decreases. The obtained oxygenation rate can be useful to control the system. In particular, if it is detected that an oxygenator provides less or decreasing oxygenation, the pump rate can be increased to increase the amount of blood passing through the oxygenator and to maintain the level of oxygen.

In some embodiments, the system further comprises at least one of a first pressure sensor and a second pressure sensor, wherein the first pressure sensor is positioned at or near the blood flow inlet and configured to measure an input pressure of the circuit and the second pressure sensor is positioned at or near the blood flow outlet and configured to measure an output pressure of the circuit, wherein the first and second blood pumps are configured to be controlled by the pump control unit such that a pump rate of the first and second blood pumps is set depending on the measured input pressure, wherein preferably the pump rates are increased when the input pressure drops to a predefined threshold. A pressure sensor at the blood flow inlet is particularly advantageous to monitor the input pressure of the system. In particular, it should be avoided to have negative pressures in the system and specifically in the pumps, which could cause gas bubbles in the blood. It is also of importance to monitor a pressure for the blood that is delivered back to the patient in order to avoid harm to the patient, e.g., by a too high output pressure.

In some embodiments, the system further comprises a third pressure sensor and a fourth pressure sensor, wherein the third pressure sensor is positioned in the first branch upstream of the first oxygenator and the fourth pressure sensor is positioned in the second branch upstream of the second oxygenator so as to detect a pressure difference, particularly a pressure drop caused by the first oxygenator and second oxygenator, respectively, by a comparison of a pressure measured by the first pressure sensor with a pressure measured by the third pressure sensor and the fourth pressure sensor, respectively. Monitoring the pressure before and after the oxygenators allows to monitor the function of the oxygenators. In particular, if an oxygenator is blocked, a pressure drop may occur (i.e., in other words, the differential pressure increases). This malfunction can then be detected easily and quickly, and the respective oxygenator can be replaced.

In some embodiments, the system further comprises at least one flow meter to measure an amount of blood flow, the at least one flow meter comprising at least one of a first flow meter and a second flow meter, the first flow meter arranged at or near the blood flow inlet and configured to measure a flow rate of blood coming from the patient, and the second flow meter arranged at or near the blood flow outlet and configured to measure a flow rate of blood that is delivered back to the patient. In particular, the incoming and outgoing flow rates can be compared to draw conclusions about any possible losses in the system. Further flow meters may be arranged in the first and second branches after the respective one of the first and second oxygenators to monitor a flow rate of blood leaving the oxygenators, particularly compared to an incoming flow rate of blood measured by the first flow meter. It will be appreciated that various flow meters may be provided throughout the system, for instance also at locations where the pressure sensors are arranged. Measurement data of a pressure sensor together with measurement data of a flow meter provide detailed information about the condition and function of the system.

The system may be considered to have a pressure feedback system particularly to control pump suction force of the blood pumps. The pump-oxygenator system of the present invention may be provided with a pressure feedback system to control the pump suction force also to eliminate the need for separate reservoirs. In this regard, the system may be a relatively static-volume system as compared to conventional systems, which normally include one or more separate reservoirs or the like. The pump-oxygenator system may be equipped with an electric heater and thermal insulation so as to eliminate the need for a heat exchanger to maintain fluid temperature. The reduced size of the system and the reduced time of the blood outside the patient's body may also eliminate the need for a heat exchange system. It will be appreciated that a heat exchange system may be nevertheless provided.

In some embodiments, the system further comprises a bubble trap at or near the blood flow outlet, wherein the bubble trap is configured to receive any blood before it is delivered back to the patient through the blood flow outlet and to retain any gas bubbles before the blood leaves the bubble trap.

In some embodiments, the first blood pump and the first oxygenator are configured as a first integrated unit, and the second blood pump and the second oxygenator are configured as a second integrated unit. The integrated units may also comprise the aforementioned valves and/or flow meters. The provision of integrated units allows for easy handling, such as installing, removing or replacing of an oxygenator along with its associated pump and possibly valve and flow meters. The integrated units may also be referred to as "sets", "modules" or "cartridges".

According to another aspect, a carrying arrangement is provided that is configured for carrying the above-described blood pump-oxygenator system. As mentioned above, the system can have a compact design to improve mobility of a patient. By providing the carrying arrangement, mobility of the patient can be further improved. The carrying arrangement can be, e.g., a trolley or other stand that has advantageously wheels to allow the patient to move around. The carrying arrangement can also be part of a wheelchair or attached to a wheelchair, such as an electric wheelchair, where the system can be placed, e.g., behind the patient. The carrying arrangement may further comprise an integrated locking mechanism that is configured to connect to the blood flow inlet, the blood flow outlet, an oxygen inlet, an oxygen outlet and an electric power port of the blood pump-oxygenator system. This allows easy placement of the system on the carrying arrangement, e.g., when it is moved from a bedside to the carrying arrangement. The system can simply be snapped into the locking mechanism thereby establishing all necessary connections at once without the need of connecting various plugs separately.

In any of the above-described systems, pump operation could be cyclically operated in timed relation to patient's heartbeat or one of the physiological parameters, and the pump control unit may vary the velocity of the pump function in response to changes in the said physiological parameter. The device itself, controller, drive line and power supply can be put on a carrying arrangement so that the patient can be ambulatory. This may significantly improve mobility of the patient compared to bulky stationary devices.

With regards to the access to the patient's blood circulation, any of the above-described systems can be used in standard ECMO procedures, i.e., no specific instruments, access, introducers, etc. are required. For instance, suitable cannulas can be placed percutaneously in desired blood vessels, such as veins and/or arteries, by the Seldinger technique, or via surgical cutdown.

It will be appreciated that explanations, embodiments, and advantages described above in connection with one aspect may similarly apply to any other aspect of the present disclosure.

### BRIEF DESCRIPTION OF THE DRAWINGS

Illustrative embodiments are described below with reference to the drawings. In the interest of clarity, not all features of an actual implementation may be described in this specification. It will of course be appreciated that in the development of any such actual embodiment, numerous implementation-specific decisions might be made to achieve the developers' specific goals, such as compliance with system-related and business-related constraints, which will vary from one implementation to another. In the drawings:
Fig. 1 shows an embodiment of a pump-oxygenator system having two oxygenators and two pumps, wherein each pump is associated with one of the oxygenators;
Fig. 2 shows an embodiment of a pump-oxygenator system having two oxygenators and two pumps as well as a reservoir, wherein each pump is associated with one of the oxygenators;
Fig. 3 shows an embodiment of a pump-oxygenator system having two oxygenators and two pumps as well as a reservoir, wherein each pump is associated with one of the oxygenators and the oxygenators and pumps are provided in combined sets;
Fig. 4 shows a diagram of the motor power of the blood pumps of the embodiment of Fig. 3;
Fig. 5 shows a connection for a pump-oxygenator set of the embodiment of Fig. 3;
Fig. 6 shows a trolley for carrying a blood pump-oxygenator system;
Fig. 7 shows a blood pump-oxygenator system connected to a console;
Fig. 8 shows a wheelchair towing a trolley with a pump-oxygenator system;
Fig. 9 shows an embodiment of a reservoir having a ball shape and multiple vents; and
Fig. 10 shows an embodiment of a reservoir having a mushroom shape and multiple vents.

### DESCRIPTION OF EMBODIMENTS

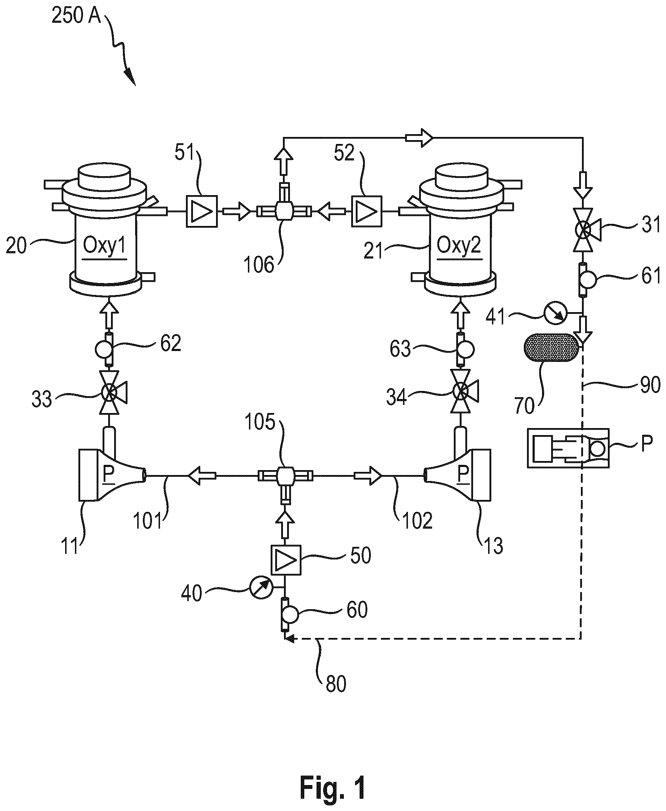

Figs. 1 to 3 show embodiments of (extracorporeal) blood pump-oxygenator systems 250A, 250B, 250C (hereinafter simply referred to as "systems") that provide a circuit between a blood flow inlet 80 and a blood flow outlet 90 with two parallel branches 101, 102 with an oxygenator 20, 21 and a blood pump 11, 13 in each of the branches. In other words, the systems 250A, 250B, 250C each comprise two oxygenators 20, 21 and two blood pumps 11, 13.

One aspect of the pump-oxygenator system 250A, 250B, 250C having two oxygenators 20, 21 (and two pumps 11, 13) is the capability of being able to replace worn oxygenators without stopping the system operation. This eliminates one of the main drawbacks of many known systems, which required the entire system to be stopped in order to replace even one element. This, in turn, required subsequent re-priming of the system after the replacement procedure had taken place. In order to be able to replace an oxygenator 20, 21, a switch may be provided that allows selection of one or more branches 101, 102 and, thus, respective oxygenators 20, 21, while disabling an oxygenator that is to be replaced. This "switch out" capability of the system disclosed herein is a significant improvement in that component replacement may be frequently required due to fast degradation of oxygenator elements.

Apart from that, a system having more than one oxygenator is provided with a redundancy to avoid a complete system failure if one oxygenator fails. Furthermore, each of the oxygenators 20, 21 can have a smaller design compared to a system having only one oxygenator. The dwell time of the blood in the oxygenators 20, 21 can be increased. This is also further supported by providing two pumps 11, 13, where each of the pumps 11, 13 is configured to feed one of the oxygenators 20, 21. The systems 250A, 250B, 250C improve safety for the patient as they provide more redundancy and increased safety when changing a pump. Each branch 101, 102 provides full functionality and can continue working while components in the other branch can be replaced.

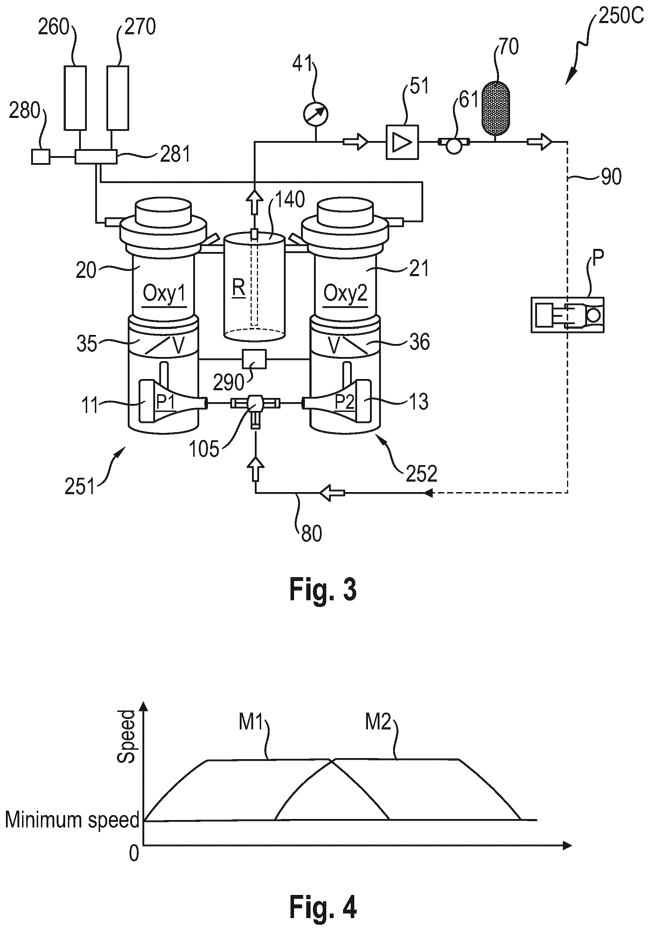

The two branches 101, 102 with the oxygenators 20, 21 and the blood pumps 11, 13 are connected to the blood flow inlet 80 and the blood flow outlet 90. Thus, the blood flows through each of the oxygenators 20, 21 when conveyed from and to the patient P through the systems 250A, 250B, 250C. The dwell time of the blood within each of the oxygenators 20, 21 can be increased by controlling the flow through the branches 101, 102 in a coordinated manner. More specifically, the control may be implemented by means of a pump control unit as described above with respect to Fig. 4, wherein the pumps 11, 13 are controlled in a counteracting manner.

Blood is drawn from a patient P and enters the system 250A, 250B, 250C at the blood flow inlet 80 and is delivered back to the patient P at the blood flow outlet 90. The blood flow inlet 80 and blood flow outlet 90 may be provided by means of an inflow cannula 80 and an outflow cannula 90, respectively. Cannulas 80, 90 may be any suitable cannula of known construction for use in transporting blood. Cannulas 80, 90 may be made from any suitable biocompatible material, including but not limited to urethane or silicone or similar biocompatible material. Cannulas 80, 90 may be reinforced with metallic wire or similar material to help the cannula resist kinking when bent in a sharp radius. All these details are well known in the cannula manufacturing industry and are included here for clarity.

The blood pumps 11, 13 may be provided (by way of example only) as a rotary or a displacement pump and, more specifically, a rotary pump of the centrifugal type. Centrifugal pumps are a known art in the blood pumping area and are preferred due to the low cost of manufacturing and the low trauma to the blood. Blood pumps 11, 13 may be directly driven by an electric motor or driven by a flexible cable that links the pump to the electric motor (as described, for example, in US 4,944,722). The coupling between the pump and the driving mechanics may be magnetically or directly coupled. The pumps 11, 13 are shown of the centrifugal type with a rotor situated inside a pump housing. The pump may be driven by a drive cable inside a sheath coupled to the rotor (not shown). The rotation of the drive cable, by an electric motor (not shown), causes the rotation of the rotor and the pumping of fluid (blood) through the pump housing. In this fashion, fluid is transported from inflow cannula 80 toward outflow cannula 90. Specifically, the pump may create a suction force that draws blood from the patient P into the system 1 through the inflow cannula 80 and that conveys the blood from the inflow cannula 80 to the outflow cannula 90.

In essence, the system is designed to drain venous blood and infuse oxygenated blood during heart diastole and to convey the blood past the oxygenator during heart systole. This will advantageously result in the heart pumping against lower systemic pressure, which translates into a lower workload on the myocardium.

The systems 250A, 250B, 250C may further comprise a bubble trap 70 at the blood flow outlet. All blood is emptied into the bubble trap 70 before it leaves to outflow cannula 90. The bubble trap 70 serves to remove any air bubbles from the pumped blood. Bubble trap 70 may include a venting port (not shown) to vent any air entrapped in bubble trap 70. Measures may be provided to increase the dwell time of the pumped blood in the bubble trap 70 to separate smaller air bubbles from the pumped blood. Bubble trap 70 may also include a screen (not shown) to trap any debris from the pumped blood before it is delivered back to the patient P.

A method of using the pump-oxygenator system described herein for (by way of example only) treating heart failure, will be described as follows:
In order to install the system, a patient's vein is percutaneously cannulated with the inflow cannula 80. The inflow cannula 80 may be provided in many different diameters and lengths to suite different patients and different applications. The Seldinger technique may be used to insert the cannula 80 into the femoral vein to drain venous blood into the circuit.

The inflow cannula 80 may then be connected to the circuit of choice. Due to the small volume of the entire circuit, blood may be used to prime the circuit. Usually, saline is used to prime standard cardiopulmonary circuits because of their large volume. Standard circuits volume may range from 100 cc (cubic centimeter; cm³) upward to 1500 cc. The circuit volume of the described system may be well below 100 cc and possible down to 10 cc. The difference in prime volume is a key factor that allows the use of blood to prime the circuit instead of saline. Using patient's blood to prime the system will eliminate the dilution the patient's blood suffers when saline is used. Diluting the patient's blood with saline may reduce the oxygen carrying capacity of the blood such that a higher circulation level is needed to achieve the same tissue oxygenation level.

The outflow cannula 90 may be inserted in the patient in a similar manner as that employed to insert the inflow cannula 80 (i.e., by using the Seldinger technique). The outflow cannula 90 may preferably be primed with the patient's blood. In case the outflow cannula 90 is placed in an artery, a pair of hemostats may be used to control blood flow through the outflow cannula 90.

The primed circuit may then be attached to the primed outflow cannula 90 while keeping the outflow cannula 90 clamped to control flow to or from outflow cannula 90. All cannulas (inflow cannula 80 and/or outflow cannula 90) may be equipped with a quick-connect for quick and ease of attaching the cannula to the circuit. Large amounts of air may be removed at this point through any port such a port of the bubble trap 70.

The circuit has a bifurcation 105 where the circuit diverts into the parallel branches 101, 102. The bifurcation 105 may be configured as a simple T- or Y-connectors. Respective valves, such as safety-type valves, may be included in the bifurcation 105 that are open during normal operation and closed, e.g., if one of the branches 101, 102 is disconnected for replacement of the respective oxygenator 20, 21. At the end of the branches 101, 102 a junction 106 is provided where the branches 101, 102 reunite leading back into a common branch of the circuit. While one or both of the bifurcation 105 and the junction 106 may be configured as such simple valves as shown in Figs. 1 to 3, one or both of the bifurcation 105 and the junction 106 may be configured as multi-way control valves (not shown). Varying a flow ratio between the branches 101, 102 can in any case be performed by controlling the blood pumps 11, 13.

Generally, a multi-way control valve has multiple ports. For instance, a three-way control valve has three ports. They may also be referred to as selector valves and be configured to act as mixing valves or as diverting valves. A diverting valve may be provided at the bifurcation 105 configured to distribute the incoming volume flow to the two branches 101, 102. A mixing valve may be provided at the junction 106 configured to mix the blood coming from the branches 101, 102. These selector valves may be configured to select one oxygenator 20, 21 or the other at a time (and, thus, may be considered acting as switches). One possibility would be to select one of the branches 101, 102, and thus one of oxygenators 20 ,21 and to block the other one. Another possibility would be to fill one oxygenator while its output is closed, and at the same time emptying the other into the patient.

In particular in combination with the pump control, where the valves may be synchronized with the pumps 11, 13, this procedure can increase the dwell time of blood inside each oxygenator 20, 21 (it can be from 3 to 200 seconds before any blood clotting). Therefore, the oxygen volume could be decreased and still attain a similar oxygenation level due to an increase in dwell time. Using this method, the whole circuit can act as a pulsatile system that changes the blood flow inside the oxygenators with each cycle of filling and emptying. In addition, the increase in dwell time will allow for better bubble detection and removal.

In the embodiment of a blood pump oxygenator system 250A shown in Fig. 1 each branch 101, 102 comprises a flow meter 62, 63 before each oxygenator 20, 21 and an oxygen sensor 51, 52 after each oxygenator 20, 21. This configuration allows to provide an integrated blood pump-oxygenator unit (also referrable to as a "set"; see also in particular Fig. 3) in each branch 101, 102, each unit comprising a pump, flow meter, oxygenator and oxygen sensor, as described in more detail below. Another oxygen sensor 50 is placed at or near the blood flow inlet 80 to be able to monitor an oxygenation rate of each of the oxygenators 20, 21 by a comparison with measurements from the respective one of the oxygen sensors 51, 52. Respective valves 33, 34 are provided in each of the branches 101, 102, which may also be part of the integrated units. It is also to be noted that it is preferred to arrange the pumps 11, 13 before the oxygenators 20, 21 so as to push the blood through the oxygenators 20, 21 rather than suction of the blood out of the oxygenators 20, 21. This can reduce the risk of gas bubbles.

A first pressure sensor 40 is located at the blood flow inlet 80 to detect an input pressure of the system 250A. For proper function, it is important that no high negative pressure occurs in the system 250A, and specifically in the pumps 11, 13, as the blood will then tend to evaporate, which may cause gas bubbles. The pumps 11, 13 may be controlled depending on the measured input pressure. A second pressure sensor 41 is located at the blood flow outlet 90 to measure an output pressure of the blood that is delivered back to the patient P. Specifically, the pressure of the blood that is delivered into the patient's veins must not be too high. A flow meter 61 may also be provided near this pressure sensor 41 to obtain data of the amount of blood that leaves the system 250A (e.g., provided in liters per minute (L/min)).

Oxygen sensors 50, 51, 52 are provided, one at or near the blood flow inlet 80 and one in each branch 101, 102 downstream the respective oxygenator 20, 21. By comparing the measured oxygen levels, more specifically the oxygen level of the blood before entering the oxygenators 20, 21 and the oxygen level of the blood after it has passed through the oxygenators 20, 21, an indication of the function of the oxygenators 20, 21 may be provided. This information may be used, e.g., to control the amount of oxygen that is fed into the oxygenators 20, 21 and/or to control the pumps 11, 13.

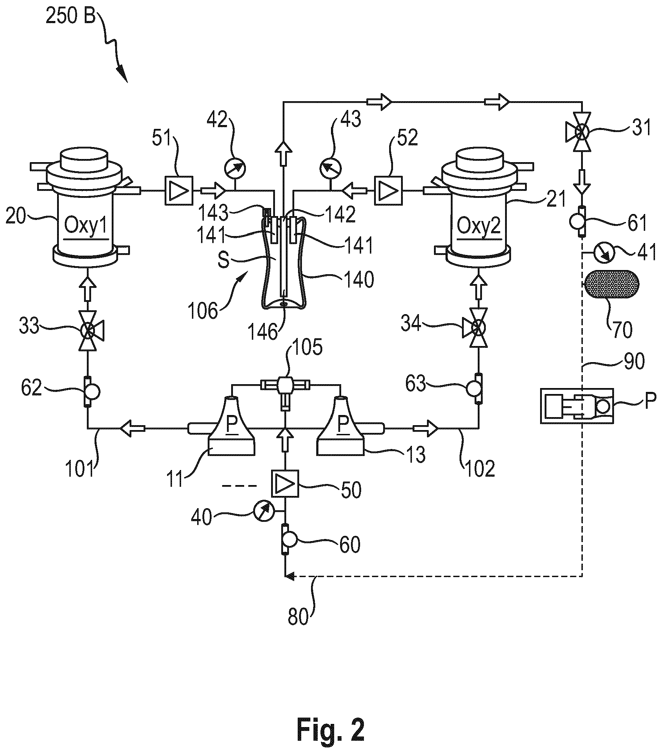

The embodiment of a blood pump oxygenator system 250B shown in Fig. 2 is similar to that described above with reference to Fig. 1. Thus, the description of Fig. 1 likewise applies to Fig. 2. A difference is that a reservoir 140 is added at the junction 106. Blood enters the reservoir 140 from all branches 101, 102 via inlet ports 141 and exits the device 140 via an outlet port 142. Gas bubbles may be exhausted at a vent 143.

As gas bubbles will move upwards, the outlet port 142 has an exit point 146 which is at a distance from the vent 143 towards the inside of the device 140. This may be achieved, e.g., by means of a tubing which extends the outlet port 142 towards the inside of the device towards a lower portion of the device 140 with an end of the tubing forming the exit point 146 for the outlet port 142. It will be appreciated that the device 140 must be in an upright (i.e., vertical, or at least substantially vertical) position in which the vent 143 is at the top of the device 140. At least, the vent 143 must be above the exit point 146 of the outlet port 142. Once the device 140 is completely filled, the occurring inner pressure will cause the blood to exit the device 140 through the exit point 146 and the outlet port 142.

The reservoir 140 may be designed with a flexible outer shell, such as a flexible bag or sack. This allows emptying the reservoir 140 by squeezing it, which reduces blood damages. The squeezing may be done either manually or by means of a mechanical squeezing device (not shown), which may, e.g., synchronized with the patient's heartbeat. The reservoir 140 may be a blood unit, wherein a heater may be provided on a back cover or lid.

Further pressure sensors 42, 43 are provided in the branches 101, 102, one upstream of each oxygenator 20, 21 and one downstream. By comparing these measured pressures, more specifically the pressure of the blood before entering the oxygenator 20 and oxygenator 21, respectively, and the pressure of the blood after it has passed through the oxygenators 20, 21, an indication of the function of the oxygenators 20, 21 may be provided. For instance, a differential pressure increase may indicate that one of the oxygenators 20, 21 is blocked.

The embodiment of a blood pump oxygenator system 250C shown in Fig. 3 is similar to that described above with reference to Fig. 2. Thus, the description of Fig. 2 likewise applies to Fig. 3. However, Fig. 3 explicitly shows a configuration with two integrated units 251, 252 that can be handled (e.g., installed, removed, replaced, etc.) as a single piece, with a reservoir 140 placed at the outputs of the two integrated units 251, 252. Each unit 251, 252 comprises a blood pump 11, 13, an oxygenator 20, 21 and a valve 35, 36 (e.g., check valves to prevent a blood flow in a backwards direction). A flow meter 62, 63 and an oxygen sensor 51, 52 (see Figs. 1 and 2) may also be included in each of the units 251, 252. A unit 251, 252 can be replaced as a whole, which may facilitate handling of the system, e.g., allows for faster switching, and may increase overall lifetime. In particular, not only the oxygenators 20, 21 may wear over time but also the blood pumps 11, 13, which is why it is advantageous to make also the blood pumps 11, 13 disposable. Respective valves, such as safety-type valves, may be included in the bifurcation 105 that are closed, e.g., if one of the pumps 11, 13 stops or is turned off to disconnect a respective one of the branches 101, 102 for maintenance or replacement. Parameters that indicate a status ("health") of the oxygenators may be provided, such as motor current of the pumps 11, 13, data from the pressure sensor 41 or the oxygenation level measured by the oxygen sensor 51. If the parameters indicate that an integrated unit 251, 252 must be replaced, an alarm can be output.

The pumps 11, 13 can be controlled in a counteracting and pulsatile manner as mentioned above, particularly by a pump control unit 290. Respective exemplary motors speeds M1, M2 of the pumps 11, 13 are shown in the diagram of Fig. 4. More specifically, while the blood flow is increased in one oxygenator 20 (motor speed of the pump 11 is increased), the blood flow in the other oxygenator 21 is slowed down (motor speed of the pump 13 is decreased), and vice versa. The curves show the motor speeds M1, M2 going up and down, respectively, between a minimum speed and a maximum speed. This allows to enhance the oxygenation for a longer period of time in one oxygenator while the other one is emptied, thereby ensuring that the blood flow to the patient is not affected.

The system 250C comprises a valve 35, 36 in each of the integrated units 251, 252 between the respective blood pump 11, 13 and the respective oxygenator 20, 21 to prevent blood from flowing in a backward direction from the oxygenator 20, 21 to the blood pump 11, 13. During the counteracting and pulsatile operation of the pumps 11, 13, which is controlled by a pump control unit 290, the pressure in the oxygenator where the blood flow is higher may push the blood in a backward direction in the other oxygenator. This can be prevented by providing the valves 35, 36, which can be configured as simple non-return valves (check valves).

The oxygenators 20, 21 are connected to an oxygen tank/concentrator 260 to enhance the blood oxygen level, and to an air compressor 270 to supply compressed (filtered ambient) air to the oxygenators 20, 21 to further remove CO₂ from the blood. A gas control unit 280 is provided to control a four-way mixing valve 281 to vary a ratio between oxygen and air and that specially allows to switch between oxygen and air that is fed to the oxygenators 20, 21. In this way, the volume of needed oxygen can be decreased such that a small oxygen tank or a portable lightweight oxygen concentrator can be used, further increasing mobility of the system 250C. The oxygenators 20, 21 can be repeatedly washed with air, supplied at a rate of, e.g., 15 L/min. The gas control unit 280 may be synchronized with the pump control unit 290. It will be appreciated that this arrangement may be applied in the systems 250A, 250B of Figs. 1 and 2, too.

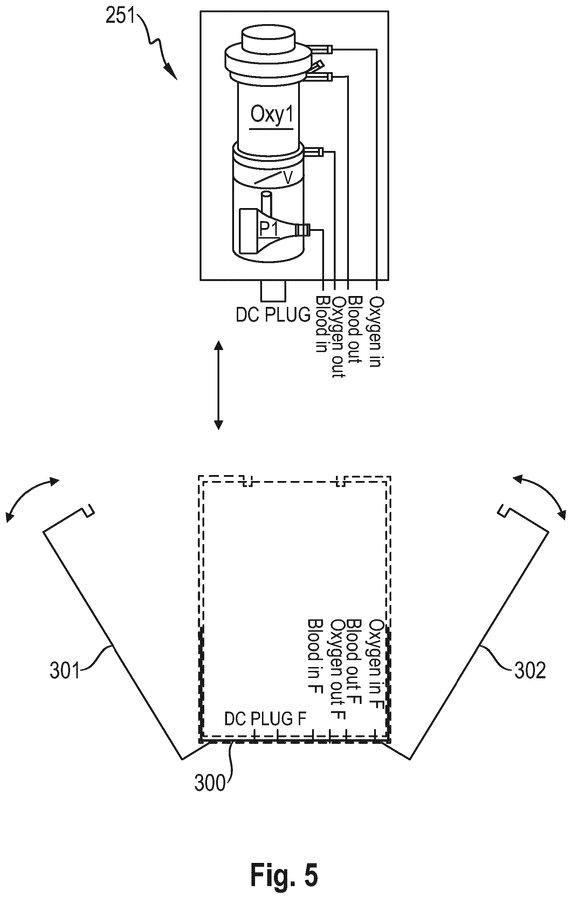

Fig. 5 schematically shows a connection for an integrated unit 251 (applies likewise to the integrated unit 252), which may be referred to as quick coupling. The various inputs and outputs (electric power (DC), blood, oxygen) may be connected and disconnected, respectively, to and from the socket 300 in one step. Locks or clamps 301, 302 may be provided that can be closed around the set 251 once it is in place in the socket 300 to securely hold it with all connections established (direction of movements upon coupling/decoupling indicated with arrows with the locked position indicated in dashed lines).

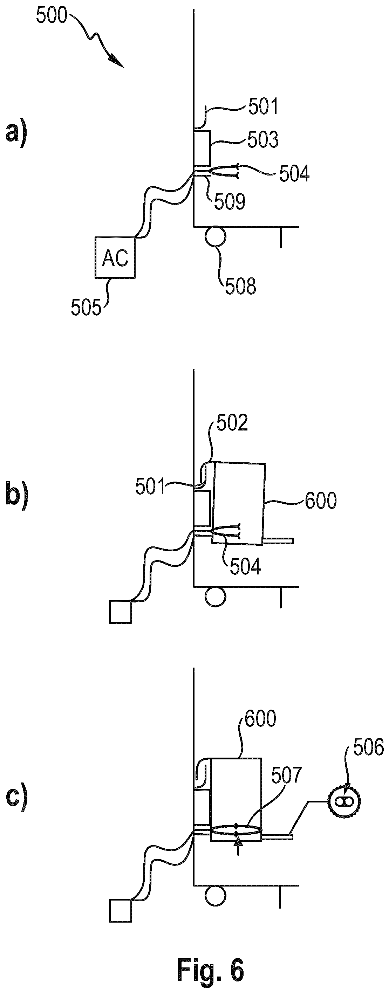

In order to provide further mobility options, a carrying arrangement in the form of a trolley 500 can be provided as shown in Fig. 6. The trolley 500 is configured to carry a blood pump-oxygenator system 600 and is shown without a system 600 placed in Fig. 6a. The system 600 can be any system described herein. The trolley 500 can be moved around using wheels 508. The trolley 500 has a carrying feature such as a hook 501 for receiving a respective hook 502 of a system 600 (see Fig. 6b showing the system 600 being placed). Fig. 6c shows the system 600 placed and locked by means of a fastening device 504, 507.

A battery of the system 600 may be charged once the system 600 is installed on the trolley 500 by means of an electric power connection 505 and a respective plug 509. A heating device, such as a ceramic heating plate 503 may be provided that can contact the system 600. Another heating option for the blood can be provided by a heat transfer between the inflow and outflow tubes arranged together in parallel (indicated at 506). An alarm can be put out if the temperature of the blood in the system 600 falls below a critical level, e.g., below 35 °C. The tubes (or hoses) that connect the system to the patient (connecting to the inflow 80 and outflow 90) may have a sufficient length such that the patient can move around while the system 600 is placed on the trolley 500. A suitable length can be, e.g., 2 to 5 m. Once the system is placed, e.g., one the trolley 500, means may be provided to roll up and stow the hoses.

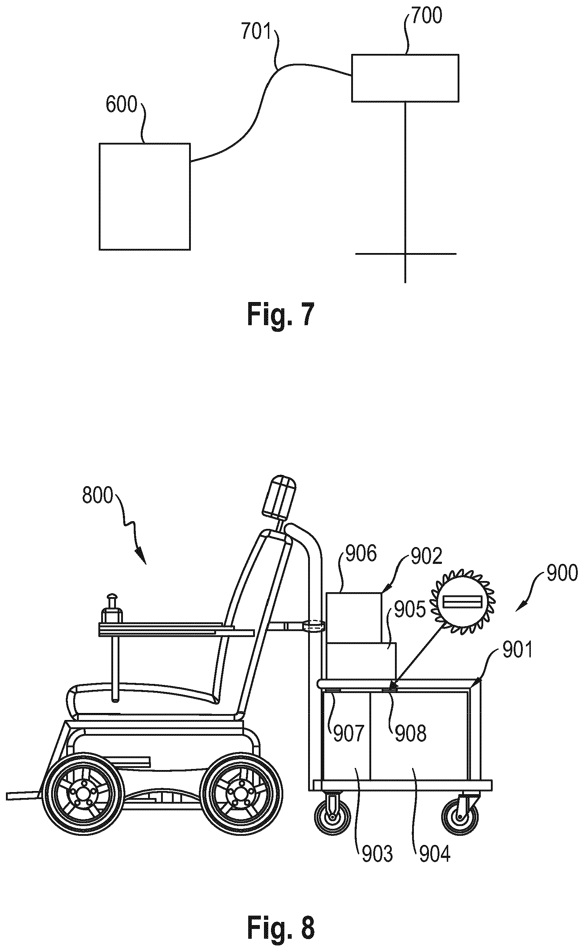

Fig. 7 schematically illustrates the system 600 being connected to a console via a cable 701. The cable 701 may be any cable such as a magnetic cable. The console 700 may be a stationary device and may provide a display, e.g., touch screen, to control the system 600, set up the system 600, read out parameters etc. It will be appreciated that the console 700 is not necessary for regular operation of the system 600 but may facilitate access to the system, e.g., by a doctor or other medical staff if necessary.

Fig. 8 shows an electrical wheelchair 800 towing a trolley 900. The rolling trolley 900 contains a fixed part 901 and a mobile part 902. The fixed part 901 is fixed to the trolley 900 and contains high-capacity batteries 903 and an oxygen concentrator 904, as well as an air pump and a power supply that should be connected to the mains when available to recharge the batteries 903 and warm up the blood circulations. The mobile part 902 includes in addition to the circuit shown specifically in Fig. 3 a small oxygen tank 905 or portable battery-operated oxygen concentrator, a small battery-operated air compressor 906, and a lightweight battery set. This mobile part 902 is connected to the fixed part via appropriate connectors 907, 908 and can be detached from the fixed part 901. It can then be carried by the patient as a backpack to serve as a standalone system for a period of time while the patient wants to move off the electric wheelchair 800. The wheelchair 800 may be a reclining chair in a way that the patient can sleep on it. In this case, the trolley 900 can be unlocked and turned to the side of or pulled back from the wheelchair 800.

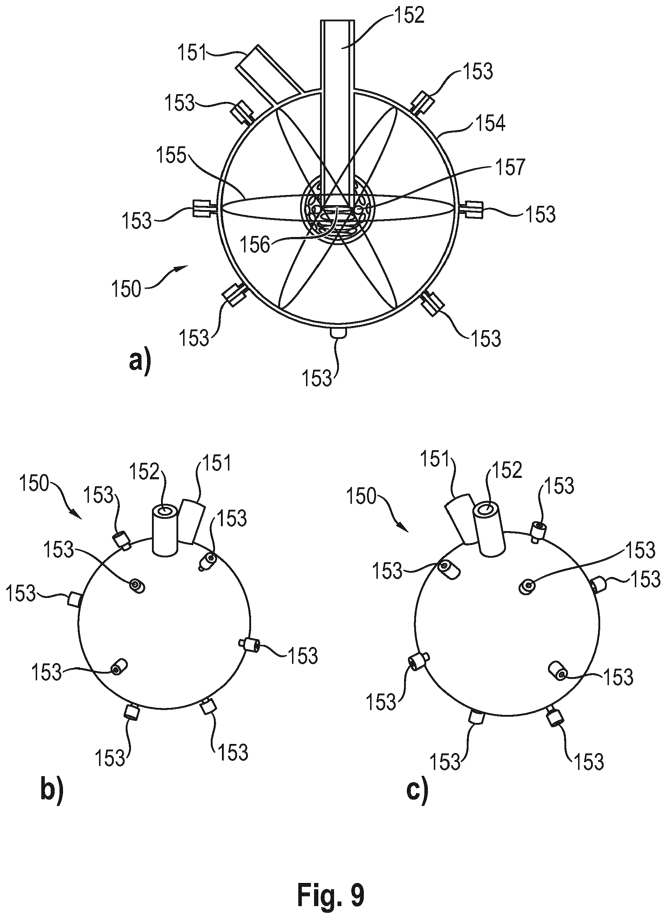

Fig. 9 shows an embodiment of a reservoir 150. It will be appreciated that it could replace the reservoir 140 of the system 10 shown in Fig. 3 (or Fig. 2). The device 150 is shown in a cross-sectional view (a) as well as in views from different angles (b, c).

The reservoir 140 described above with reference to Fig. 2 may have a flexible bag-like configuration, and must be in an upright position during operation to keep the vent oriented upwards as described above with the exit point 146 of the outlet port 143 being below the vent 143. However, particularly with regards to mobility options, the position and orientation may change as the patient moves (using the detachable unit 902). Thus, it might not always be ensured that the vent 143 of the device 140 faces upwards. The reservoir 150 shown in Fig. 9, thus, has a design which ensures proper function independent from its orientation. Apart from that, the function of the device 150 is similar to that of the device 140.

The reservoir 150 has one inlet port 151. More than one inlet port may be provided depending on the number of oxygenators of the blood pump-oxygenator system, in which the device 150 is intended to be used. An outlet port 152 is provided with an exit point 156 that is substantially in the center of the device 150. A plurality of vents 153 is provided distributed along the outer circumference of the device 150. The number and position of vents 153 may vary as long as at least one of the vents 153 can be positioned substantially at the top of the device 150 in any orientation of the device 150. This configuration ensures that in any orientation of the device 150 at least one of the vents 153 is above the exit point 156 of the outlet port such that gas bubbles can escape. In order to retain any debris, such as blood clots, and to avoid such debris to be conveyed through the system, a filter 157, such as a mesh- or sponge-like structure may be provided covering the exit point 156 of the outlet port 152.

The device 150 has a ball shape, particularly a spherical shape. It will be appreciated, however, that any shape may be envisioned as long as the described arrangement of a plurality of vents, inlet port(s) and outlet port is provided. In order to allow the device 150 to be squeezed as described above with respect to the device 140 it may have a flexible outer shell 154. A biasing arrangement, such as metallic rings 155 may be provided to allow the device 150 to return to its original shape. It will be appreciated, however, that the device 150 may alternatively have a hard outer shell. In any case, blood will be pushed out of the outlet port 142 once the device 150 is completely filled.

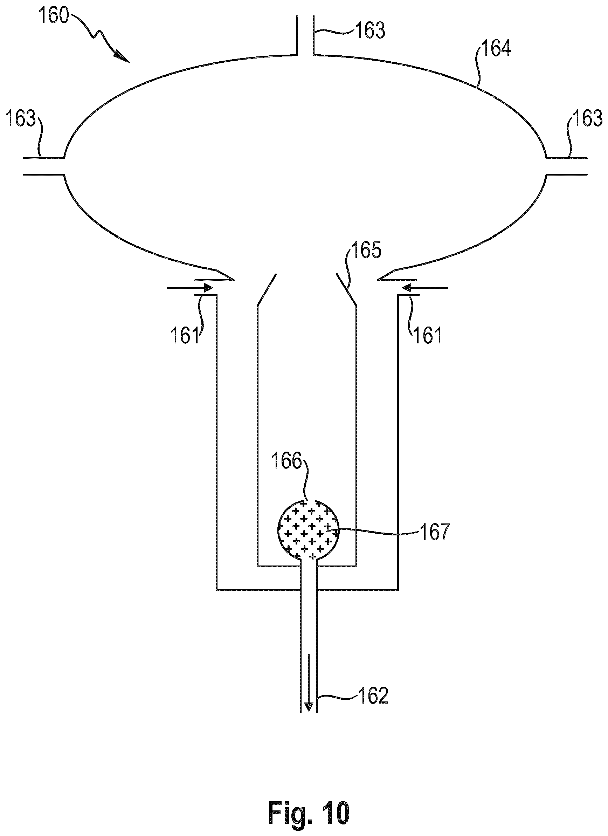

Fig. 10 shows another embodiment of a reservoir 160. It will be appreciated that the reservoir 160 may be used instead of the reservoir 140 or the reservoir 150 described above and may likewise be used in any system accordingly. Like the other reservoirs, the reservoir 160 may also function as a bubble trap as will be described below.

The reservoir 160 has two inlet ports 161 to receive blood from the oxygenators (it will be appreciated that the number of inlet ports may vary depending on the configuration of the system, more specifically the number of oxygenators). The inlet ports 161 are located in a middle section of the reservoir 160 and above an outlet port 162, in particular when the reservoir 160 is in an upright position. This configuration with an exit point 166 of the outlet port being in a lower portion of the reservoir 160 ensures that any bubbles in the blood entering the reservoir 160 will not directly reach the outlet port 162, more specifically the exit point 166 of the outlet port 162. A shield 165 surrounding the region where the exit point 166 is located is provided to direct the entering blood flow away from the exit point 166. The entering blood will be directed towards an upper portion 164 of the reservoir in which vents 163 are provided to exhaust any gas bubbles. Like the above-described ball shaped reservoir 150, the portion 164 has a rounded shape with several vents 163 at different positions to ensure that gas bubbles can leave the reservoir 160 even if it is not in a fully upright position. Gas bubbles will then escape via a respective one of the vents 163 that is in an uppermost position. In order to retain any debris, such as blood clots, and to avoid such debris to be conveyed through the system, a filter 167, such as a mesh- or sponge-like structure may be provided at the exit point 166 of the outlet port 162.

The described systems can be used in conjunction with any cardiopulmonary bypass system (CPB) or any extra corporeal membrane oxygenation (ECMO) to increase the oxygenation of blood with a smaller oxygenator size. Using a smaller oxygenator size will decrease the priming volume of the circuit, therefore, it will decrease blood dilution and increase blood capability to carry more oxygen to the patient. In addition, the system will allow the use of ambient air instead of pure oxygen and will attain a similar level of oxygenation. Eliminating the use gas with high oxygen concentration will eliminate the risk associated with flammability of concentrated oxygen and, therefore, will allow the use of the systems in environments outside hospital setting, such as ambulance and environment where highly concentrated oxygen is not available.

It will be appreciated that in accordance with any embodiment described herein, an electronic controller may be required that controls the features of the system (such as pump speed, oxygen gas volume delivery, valve opening and closing) as well as the diagnostic sensors (pressure sensors, oxygen sensors, flow meters) that indicate proper performance of the system. More specifically, the components of the system may be controlled in such a way that the system may be automatically controlled by respective feedback loops, e.g., the pump(s) and valve(s) may be controlled using sensor data from the diagnostic sensors. The specifics of such controller are common to those skilled in the art.

While above at least one exemplary embodiment of the present invention has been described, it has to be noted that a great number of variations thereto exists and that the scope of the invention is defined by the appended claims. Furthermore, it is appreciated that the described exemplary embodiments only illustrate non-limiting examples of how the present invention can be implemented and that it is not intended to limit the scope, the application or the configuration of the herein-described apparatuses and methods. Rather, the preceding description will provide the person skilled in the art with constructions for implementing at least one exemplary embodiment of the invention, wherein it has to be understood that various changes of functionality and the arrangement of the elements of the exemplary embodiment can be made, without deviating from the subject-matter defined by the appended claims and their legal equivalents.

## Claims

1. A blood pump-oxygenator system (250A; 250B, 250C) for increasing perfusion and oxygen level in a patient (P), comprising a blood flow inlet (80) and a blood flow outlet (90) which are connected so as to form a circuit to be operable as a cardiopulmonary bypass system adapted for extracorporeal processing of the patient's blood, a first blood pump (11) and a first oxygenator (20), wherein the first blood pump (11) is configured to convey blood through the circuit from the patient (P) into the blood flow inlet (80), through the first oxygenator (20) and out of the blood flow outlet (90) back into the patient (P), wherein the system further comprises:
a second blood pump (13) and a second oxygenator (21), wherein the second blood pump (13) is configured to convey blood through the circuit from the patient (P) into the blood flow inlet (80), through the second oxygenator (20) and out of the blood flow outlet (90) back into the patient (P), wherein the circuit comprises a first branch (101) and a second branch (102) arranged in parallel with each other, the first branch (101) accommodating the first blood pump (11) and the first oxygenator (20), and the second branch (102) accommodating the second blood pump (13) and the second oxygenator (21), wherein the first and second blood pumps (11, 13) are mechanical blood pumps; and
a pump control unit (290), wherein the first and second blood pumps (11, 13) are configured to be controlled by the pump control unit (290) to increase or decrease a flow rate of blood through the respective oxygenator (20, 21) in such a manner to vary a ratio of flow rates of blood through the first and second oxygenators (20, 21).

2. The blood pump-oxygenator system of claim 1, wherein the first and second blood pumps (11, 13) are configured to be controlled by the pump control unit (290) to increase or decrease the flow rate of blood through the respective oxygenator (20, 21) in a synchronized and opposite manner.

3. The blood pump-oxygenator system of claim 1 or 2, wherein the first and second blood pumps (11, 13) are configured to be controlled by the pump control unit (290) to increase or decrease the flow rate of blood through the respective oxygenator (20, 21) while a total flow rate remains at a required level.

4. The blood pump-oxygenator system of any one of the preceding claims, further comprising a gas control unit (280), wherein the gas control unit (280) is configured to control a flow of oxygen as well as a flow of air to the first and second oxygenators (20, 21) in such a manner to vary a ratio of flow rates of oxygen and air through the first and second oxygenators (20, 21), respectively.

5. The blood pump-oxygenator system of claim 4, wherein the gas control unit (280) is configured to be synchronized with the pump control unit (290).

6. The blood pump-oxygenator system of any one of the preceding claims, further comprising an oxygen concentrator (260) for the supply of oxygen to the first and second oxygenators (20, 21) and an air compressor (270) for the supply of air to the first and second oxygenators (20, 21).

7. The blood pump-oxygenator system of any one of the preceding claims, further comprising a valve (33, 34) in each of the branches (101, 102) between the respective blood pump (11, 13) and the respective oxygenator (20, 21) to prevent blood from flowing in a backward direction from the oxygenator (20, 21) to the blood pump (11, 13).

8. The blood pump-oxygenator system of any one of the preceding claims, wherein the circuit comprises a bifurcation (105) that diverts the blood flow in the circuit into the two branches (101, 102), and a junction (105) where the at least two branches (101, 102) are brought together, wherein at least one of the bifurcation (105) and the junction (106) is configured as a multi-way control valve (130, 131) that is operable so as to select at least one of the two branches (101, 102) for the blood to flow through.

9. The blood pump-oxygenator system of claim 8, wherein a reservoir (140; 150; 160) is provided at the junction (106), the reservoir (140; 150; 160) comprising at least two inlet ports (141; 151; 161), wherein each of the branches (101, 102) ends in the reservoir (140; 150; 160) at a respective inlet port (141; 151; 161), an outlet port (142; 152; 162) for the blood to exit the reservoir (140; 150; 160), and at least one vent (143; 153; 163) configured for exhausting gas bubbles in the blood in the reservoir (140; 150; 160), wherein the outlet port is extended towards an inside of the reservoir (140; 150; 160).

10. The blood pump-oxygenator system of any one of the preceding claims, further comprising a first oxygen sensor (50), a second oxygen sensor (51) and a third oxygen sensor (52), all configured to measure an oxygen level in the blood, wherein the first oxygen sensor (50) is positioned upstream of the first and second oxygenators (20, 21), wherein the second oxygen sensor (51) is positioned in the first branch (101) downstream of the first oxygenator (20) so as to obtain an oxygenation rate of the first oxygenator (20) by a comparison of an oxygen level measured by the first oxygen sensor (50) with an oxygen level measured by the second oxygen sensor (51), and wherein the third oxygen sensor (52) is positioned in the second branch (102) downstream of the second oxygenator (21) so as to obtain an oxygenation rate of the second oxygenator (21) by a comparison of an oxygen level measured by the first oxygen sensor (50) with an oxygen level measured by the third oxygen sensor (52).

11. The blood pump-oxygenator system of any one of the preceding claims, further comprising at least one of a first pressure sensor (40) and a second pressure sensor (41), wherein the first pressure sensor (40) is positioned at the blood flow inlet (80) and configured to measure an input pressure of the circuit and the second pressure sensor (41) is positioned at the blood flow outlet (90) and configured to measure an output pressure of the circuit, wherein the first and second blood pumps (11, 13) are configured to be controlled by the pump control unit (290) such that a pump rate of the first and second blood pumps (11, 13) is set depending on the measured input pressure, wherein preferably the pump rates are increased when the input pressure drops to a predefined threshold.

12. The blood pump-oxygenator system of claim 11, further comprising a third pressure sensor (42) and a fourth pressure sensor (43), wherein the third pressure sensor (42) is positioned in the first branch (101) upstream of the first oxygenator (20) and the fourth pressure sensor (43) is positioned in the second branch (102) upstream of the second oxygenator (21) so as to detect a pressure drop caused by the first oxygenator (20) and second oxygenator (21), respectively, by a comparison of a pressure measured by the first pressure sensor (40) with a pressure measured by the third pressure sensor (42) and the fourth pressure sensor (43), respectively.

13. The blood pump-oxygenator system of any one of the preceding claims, further comprising at least one flow meter to measure an amount of blood flow, the at least one flow meter comprising at least one of a first flow meter (60) and a second flow meter (61), the first flow meter (60) arranged at the blood flow inlet (80) and configured to measure a flow rate of blood coming from the patient (P), and the second flow meter (61) arranged at the blood flow outlet (90) and configured to measure a flow rate of blood that is delivered back to the patient (P).

14. The blood pump-oxygenator system of any one of the preceding claims, wherein the first blood pump (11) and the first oxygenator (20) are configured as a first integrated unit (251), and the second blood pump (13) and the second oxygenator (21) are configured as a second integrated unit (252).

15. A carrying arrangement (500; 900), configured for carrying the blood pump-oxygenator system of any one of the preceding claims that further includes an electric power port for supplying power to the pump control unit, the carrying arrangement further comprising an integrated locking mechanism that is configured to connect to the blood flow inlet (80), the blood flow outlet (90), an oxygen inlet, an oxygen outlet and the electric power port of the blood pump-oxygenator system in a single docking action.

## Patentansprüche

1. Blutpumpen-Oxygenator-System (250A; 250B, 250C) zum Erhöhen der Perfusion und des Sauerstoffgehalts in einem Patienten (P), umfassend einen Blutflusseinlass (80) und einen Blutflussauslass (90), die so verbunden sind, dass sie einen Kreislauf bilden, der als kardiopulmonales Bypass-System betrieben werden kann, das zur extrakorporalen Verarbeitung des Blutes des Patienten angepasst ist, eine erste Blutpumpe (11) und einen ersten Oxygenator (20), wobei die erste Blutpumpe (11) so konfiguriert ist, dass sie Blut durch den Kreislauf vom Patienten (P) in den Blutflusseinlass (80), durch den ersten Oxygenator (20) und aus dem Blutflussauslass (90) zurück in den Patienten (P) fördert, wobei das System ferner umfasst:
eine zweite Blutpumpe (13) und einen zweiten Oxygenator (21), wobei die zweite Blutpumpe (13) so konfiguriert ist, dass sie Blut durch den Kreislauf vom Patienten (P) in den Blutflusseinlass (80), durch den zweiten Oxygenator (20) und aus dem Blutflussauslass (90) zurück in den Patienten (P) fördert, wobei der Kreislauf einen ersten Zweig (101) und einen zweiten Zweig (102) umfasst, die parallel zueinander angeordnet sind, wobei der erste Zweig (101) die erste Blutpumpe (11) und den ersten Oxygenator (20) aufnimmt und der zweite Zweig (102) die zweite Blutpumpe (13) und den zweiten Oxygenator (21) aufnimmt, wobei die erste und die zweite Blutpumpe (11, 13) mechanische Blutpumpen sind; und
eine Pumpensteuereinheit (290), wobei die erste und die zweite Blutpumpe (11, 13) so konfiguriert sind, dass sie von der Pumpensteuereinheit (290) gesteuert werden, um eine Durchflussrate von Blut durch den jeweiligen Oxygenator (20, 21) in einer solchen Weise zu erhöhen oder zu verringern, dass ein Verhältnis von Durchflussraten von Blut durch den ersten und den zweiten Oxygenator (20, 21) variiert wird.

2. Blutpumpen-Oxygenator-System nach Anspruch 1, wobei die erste und die zweite Blutpumpe (11, 13) so konfiguriert sind, dass sie von der Pumpensteuereinheit (290) gesteuert werden, um die Durchflussrate von Blut durch den jeweiligen Oxygenator (20, 21) in einer synchronisierten und entgegengesetzten Weise zu erhöhen oder zu verringern.

3. Blutpumpen-Oxygenator-System nach Anspruch 1 oder 2, wobei die erste und die zweite Blutpumpe (11, 13) so konfiguriert sind, dass sie von der Pumpensteuereinheit (290) gesteuert werden, um die Durchflussrate von Blut durch den jeweiligen Oxygenator (20, 21) zu erhöhen oder zu verringern, während eine Gesamtdurchflussrate auf einem erforderlichen Niveau bleibt.

4. Blutpumpen-Oxygenator-System nach einem der vorhergehenden Ansprüche, ferner umfassend eine Gassteuereinheit (280), wobei die Gassteuereinheit (280) so konfiguriert ist, dass sie einen Sauerstoffstrom sowie einen Luftstrom zu dem ersten und dem zweiten Oxygenator (20, 21) in einer solchen Weise steuert, dass ein Verhältnis von Durchflussraten von Sauerstoff und Luft durch den ersten bzw. den zweiten Oxygenator (20, 21) variiert wird.

5. Blutpumpen-Oxygenator-System nach Anspruch 4, wobei die Gassteuereinheit (280) so konfiguriert ist, dass sie mit der Pumpensteuereinheit (290) synchronisiert ist.

6. Blutpumpen-Oxygenator-System nach einem der vorhergehenden Ansprüche, ferner umfassend einen Sauerstoffkonzentrator (260) für die Zufuhr von Sauerstoff zu dem ersten und dem zweiten Oxygenator (20, 21) und einen Luftkompressor (270) für die Zufuhr von Luft zu dem ersten und dem zweiten Oxygenator (20, 21).

7. Blutpumpen-Oxygenator-System nach einem der vorhergehenden Ansprüche, ferner umfassend ein Ventil (33, 34) in jedem der Zweige (101, 102) zwischen der jeweiligen Blutpumpe (11, 13) und dem jeweiligen Oxygenator (20, 21), um zu verhindern, dass Blut in einer Rückwärtsrichtung von dem Oxygenator (20, 21) zu der Blutpumpe (11, 13) fließt.

8. Blutpumpen-Oxygenator-System nach einem der vorhergehenden Ansprüche, wobei der Kreislauf eine Verzweigung (105), die den Blutstrom in dem Kreislauf in die zwei Zweige (101, 102) umleitet, und eine Verbindungsstelle (105), an der die mindestens zwei Zweige (101, 102) zusammengebracht werden, umfasst, wobei mindestens eine von der Verzweigung (105) und der Verbindungsstelle (106) als ein Mehrwege-Steuerventil (130, 131) konfiguriert ist, das so betreibbar ist, dass es mindestens einen der zwei Zweige (101, 102) auswählt, durch den das Blut fließen kann.

9. Blutpumpen-Oxygenator-System nach Anspruch 8, wobei ein Reservoir (140; 150; 160) an der Verbindungsstelle (106) bereitgestellt ist, wobei das Reservoir (140; 150; 160) mindestens zwei Einlassöffnungen (141; 151; 161), wobei jeder der Zweige (101, 102) in dem Reservoir (140; 150; 160) an einer jeweiligen Einlassöffnung (141; 151; 161) endet, eine Auslassöffnung (142; 152; 162), damit das Blut aus dem Reservoir (140; 150; 160) austreten kann, und mindestens eine Entlüftung (143; 153; 163), die zum Ablassen von Gasblasen in dem Blut in dem Reservoir (140; 150; 160) konfiguriert ist, umfasst, wobei sich die Auslassöffnung in Richtung eines Inneren des Reservoirs (140; 150; 160) erstreckt.

10. Blutpumpen-Oxygenator-System nach einem der vorhergehenden Ansprüche, ferner umfassend einen ersten Sauerstoffsensor (50), einen zweiten Sauerstoffsensor (51) und einen dritten Sauerstoffsensor (52), die alle so konfiguriert sind, dass sie einen Sauerstoffgehalt in dem Blut messen, wobei der erste Sauerstoffsensor (50) stromaufwärts des ersten und des zweiten Oxygenators (20, 21) positioniert ist, wobei der zweite Sauerstoffsensor (51) in dem ersten Zweig (101) stromabwärts des ersten Oxygenators (20) positioniert ist, um eine Oxygenierungsrate des ersten Oxygenators (20) durch einen Vergleich eines von dem ersten Sauerstoffsensor (50) gemessenen Sauerstoffgehalts mit einem von dem zweiten Sauerstoffsensor (51) gemessenen Sauerstoffgehalt zu erhalten, und wobei der dritte Sauerstoffsensor (52) in dem zweiten Zweig (102) stromabwärts des zweiten Oxygenators (21) positioniert ist, um eine Oxygenierungsrate des zweiten Oxygenators (21) durch einen Vergleich eines von dem ersten Sauerstoffsensor (50) gemessenen Sauerstoffgehalts mit einem von dem dritten Sauerstoffsensor (52) gemessenen Sauerstoffgehalt zu erhalten.

11. Blutpumpen-Oxygenator-System nach einem der vorhergehenden Ansprüche, ferner umfassend mindestens einen von einem ersten Drucksensor (40) und einem zweiten Drucksensor (41), wobei der erste Drucksensor (40) an dem Blutflusseinlass (80) positioniert und so konfiguriert ist, dass er einen Eingangsdruck des Kreislaufs misst, und der zweite Drucksensor (41) an dem Blutflussauslass (90) positioniert und so konfiguriert ist, dass er einen Ausgangsdruck des Kreislaufs misst, wobei die erste und die zweite Blutpumpe (11, 13) so konfiguriert sind, dass sie von der Pumpensteuereinheit (290) so gesteuert werden, dass eine Pumpenrate der ersten und der zweiten Blutpumpe (11, 13) in Abhängigkeit von dem gemessenen Eingangsdruck eingestellt wird, wobei vorzugsweise die Pumpenraten erhöht werden, wenn der Eingangsdruck auf einen vordefinierten Schwellenwert fällt.

12. Blutpumpen-Oxygenator-System nach Anspruch 11, ferner umfassend einen dritten Drucksensor (42) und einen vierten Drucksensor (43), wobei der dritte Drucksensor (42) in dem ersten Zweig (101) stromaufwärts des ersten Oxygenators (20) positioniert ist und der vierte Drucksensor (43) in dem zweiten Zweig (102) stromaufwärts des zweiten Oxygenators (21) positioniert ist, um einen Druckabfall, der durch den ersten Oxygenator (20) bzw. den zweiten Oxygenator (21) verursacht wird, durch einen Vergleich eines von dem ersten Drucksensor (40) gemessenen Drucks mit einem von dem dritten Drucksensor (42) bzw. dem vierten Drucksensor (43) gemessenen Druck zu erfassen.

13. Blutpumpen-Oxygenator-System nach einem der vorhergehenden Ansprüche, ferner umfassend mindestens einen Durchflussmesser zum Messen einer Menge an Blutfluss, wobei der mindestens eine Durchflussmesser mindestens einen von einem ersten Durchflussmesser (60) und einem zweiten Durchflussmesser (61) umfasst, wobei der erste Durchflussmesser (60) an dem Blutflusseinlass (80) angeordnet und so konfiguriert ist, dass er eine Durchflussrate von Blut misst, das von dem Patienten (P) kommt, und der zweite Durchflussmesser (61) an dem Blutflussauslass (90) angeordnet und so konfiguriert ist, dass er eine Durchflussrate von Blut misst, das dem Patienten (P) zurückgegeben wird.

14. Blutpumpen-Oxygenator-System nach einem der vorhergehenden Ansprüche, wobei die erste Blutpumpe (11) und der erste Oxygenator (20) als eine erste integrierte Einheit (251) konfiguriert sind und die zweite Blutpumpe (13) und der zweite Oxygenator (21) als eine zweite integrierte Einheit (252) konfiguriert sind.

15. Trageanordnung (500; 900), die zum Tragen des Blutpumpen-Oxygenator-Systems nach einem der vorhergehenden Ansprüche konfiguriert ist und ferner einen elektrischen Stromanschluss zur Stromversorgung der Pumpensteuereinheit umfasst, wobei die Trageanordnung ferner einen integrierten Verriegelungsmechanismus umfasst, der so konfiguriert ist, dass er mit dem Blutflusseinlass (80), dem Blutflussauslass (90), einem Sauerstoffeinlass, einem Sauerstoffauslass und dem elektrischen Stromanschluss des Blutpumpen-Oxygenator-Systems in einer einzigen Andockaktion verbunden ist.

## Revendications

1. Système de pompe à sang-oxygénateur (250A ; 250B, 250C) pour augmenter la perfusion et le niveau d'oxygène chez un patient (P), comprenant une entrée de flux sanguin (80) et une sortie de flux sanguin (90) qui sont reliées de manière à former un circuit pouvant fonctionner comme un système de dérivation cardiopulmonaire adapté pour le traitement extracorporel du sang du patient, une première pompe à sang (11) et un premier oxygénateur (20), dans lequel la première pompe à sang (11) est configurée pour transporter le sang à travers le circuit du patient (P) dans l'entrée de flux sanguin (80), à travers le premier oxygénateur (20) et hors de la sortie de flux sanguin (90) de retour dans le patient (P), dans lequel le système comprend en outre :
une seconde pompe à sang (13) et un second oxygénateur (21), dans lequel la seconde pompe à sang (13) est configurée pour transporter le sang à travers le circuit du patient (P) dans l'entrée de flux sanguin (80), à travers le second oxygénateur (20) et hors de la sortie de flux sanguin (90) de retour dans le patient (P), dans lequel le circuit comprend une première branche (101) et une seconde branche (102) disposées en parallèle l'une à l'autre, la première branche (101) recevant la première pompe à sang (11) et le premier oxygénateur (20), et la seconde branche (102) recevant la seconde pompe à sang (13) et le second oxygénateur (21), dans lequel les première et seconde pompes à sang (11, 13) sont des pompes à sang mécaniques ; et
une unité de commande de pompe (290), dans lequel les première et seconde pompes à sang (11, 13) sont configurées pour être commandées par l'unité de commande de pompe (290) pour augmenter ou diminuer un débit de sang à travers l'oxygénateur respectif (20, 21) de manière à faire varier un rapport des débits de sang à travers les premier et second oxygénateurs (20, 21).

2. Système de pompe à sang-oxygénateur selon la revendication 1, dans lequel les première et seconde pompes à sang (11, 13) sont configurées pour être commandées par l'unité de commande de pompe (290) pour augmenter ou diminuer le débit de sang à travers l'oxygénateur respectif (20, 21) de manière synchronisée et opposée.

3. Système de pompe à sang-oxygénateur selon la revendication 1 ou 2, dans lequel les première et seconde pompes à sang (11, 13) sont configurées pour être commandées par l'unité de commande de pompe (290) pour augmenter ou diminuer le débit de sang à travers l'oxygénateur respectif (20, 21) tandis qu'un débit total reste à un niveau requis.

4. Système de pompe à sang-oxygénateur selon l'une quelconque des revendications précédentes, comprenant en outre une unité de commande de gaz (280), dans lequel l'unité de commande de gaz (280) est configurée pour commander un flux d'oxygène ainsi qu'un flux d'air vers les premier et second oxygénateurs (20, 21) de manière à faire varier un rapport des débits d'oxygène et d'air à travers les premier et second oxygénateurs (20, 21), respectivement.

5. Système de pompe à sang-oxygénateur selon la revendication 4, dans lequel l'unité de commande de gaz (280) est configurée pour être synchronisée avec l'unité de commande de pompe (290).

6. Système de pompe à sang-oxygénateur selon l'une quelconque des revendications précédentes, comprenant en outre un concentrateur d'oxygène (260) pour l'alimentation en oxygène des premier et second oxygénateurs (20, 21) et un compresseur d'air (270) pour l'alimentation en air des premier et second oxygénateurs (20, 21).

7. Système de pompe à sang-oxygénateur selon l'une quelconque des revendications précédentes, comprenant en outre une vanne (33, 34) dans chacune des branches (101, 102) entre la pompe à sang respective (11, 13) et l'oxygénateur respectif (20, 21) pour empêcher le sang de s'écouler dans une direction arrière de l'oxygénateur (20, 21) à la pompe à sang (11, 13).

8. Système de pompe à sang-oxygénateur selon l'une quelconque des revendications précédentes, dans lequel le circuit comprend une bifurcation (105) qui dévie le flux sanguin dans le circuit dans les deux branches (101, 102), et une jonction (105) où les au moins deux branches (101, 102) sont réunies, dans lequel au moins l'une de la bifurcation (105) et de la jonction (106) est configurée comme une vanne de commande à voies multiples (130, 131) qui peut fonctionner de manière à sélectionner au moins l'une des deux branches (101, 102) pour que le sang s'écoule à travers celle-ci.

9. Système de pompe à sang-oxygénateur selon la revendication 8, dans lequel un réservoir (140 ; 150 ; 160) est prévu au niveau de la jonction (106), le réservoir (140 ; 150 ; 160) comprenant au moins deux orifices d'entrée (141 ; 151 ; 161), dans lequel chacune des branches (101, 102) se termine dans le réservoir (140 ; 150 ; 160) au niveau d'un orifice d'entrée respectif (141 ; 151 ; 161), un orifice de sortie (142 ; 152 ; 162) pour que le sang sorte du réservoir (140 ; 150 ; 160), et au moins un évent (143 ; 153 ; 163) configuré pour évacuer des bulles de gaz dans le sang dans le réservoir (140 ; 150 ; 160), dans lequel l'orifice de sortie s'étend vers un intérieur du réservoir (140 ; 150 ; 160).

10. Système de pompe à sang-oxygénateur selon l'une quelconque des revendications précédentes, comprenant en outre un premier capteur d'oxygène (50), un deuxième capteur d'oxygène (51) et un troisième capteur d'oxygène (52), tous configurés pour mesurer un niveau d'oxygène dans le sang, dans lequel le premier capteur d'oxygène (50) est positionné en amont des premier et second oxygénateurs (20, 21), dans lequel le deuxième capteur d'oxygène (51) est positionné dans la première branche (101) en aval du premier oxygénateur (20) de manière à obtenir un taux d'oxygénation du premier oxygénateur (20) par une comparaison d'un niveau d'oxygène mesuré par le premier capteur d'oxygène (50) avec un niveau d'oxygène mesuré par le deuxième capteur d'oxygène (51), et dans lequel le troisième capteur d'oxygène (52) est positionné dans la seconde branche (102) en aval du second oxygénateur (21) de manière à obtenir un taux d'oxygénation du second oxygénateur (21) par une comparaison d'un niveau d'oxygène mesuré par le premier capteur d'oxygène (50) avec un niveau d'oxygène mesuré par le troisième capteur d'oxygène (52).

11. Système de pompe à sang-oxygénateur selon l'une quelconque des revendications précédentes, comprenant en outre au moins l'un d'un premier capteur de pression (40) et d'un deuxième capteur de pression (41), dans lequel le premier capteur de pression (40) est positionné au niveau de l'entrée de flux sanguin (80) et configuré pour mesurer une pression d'entrée du circuit et le deuxième capteur de pression (41) est positionné au niveau de la sortie de flux sanguin (90) et configuré pour mesurer une pression de sortie du circuit, dans lequel les première et seconde pompes à sang (11, 13) sont configurées pour être commandées par l'unité de commande de pompe (290) de telle sorte qu'un débit de pompe des première et seconde pompes à sang (11, 13) est réglé en fonction de la pression d'entrée mesurée, dans lequel de préférence les débits de pompe sont augmentés lorsque la pression d'entrée chute à un seuil prédéfini.

12. Système de pompe à sang-oxygénateur selon la revendication 11, comprenant en outre un troisième capteur de pression (42) et un quatrième capteur de pression (43), dans lequel le troisième capteur de pression (42) est positionné dans la première branche (101) en amont du premier oxygénateur (20) et le quatrième capteur de pression (43) est positionné dans la seconde branche (102) en amont du second oxygénateur (21) de manière à détecter une chute de pression provoquée par le premier oxygénateur (20) et le second oxygénateur (21), respectivement, par une comparaison d'une pression mesurée par le premier capteur de pression (40) avec une pression mesurée par le troisième capteur de pression (42) et le quatrième capteur de pression (43), respectivement.

13. Système de pompe à sang-oxygénateur selon l'une quelconque des revendications précédentes, comprenant en outre au moins un débitmètre pour mesurer une quantité de flux sanguin, l'au moins un débitmètre comprenant au moins l'un d'un premier débitmètre (60) et d'un deuxième débitmètre (61), le premier débitmètre (60) étant disposé au niveau de l'entrée de flux sanguin (80) et configuré pour mesurer un débit de sang provenant du patient (P), et le deuxième débitmètre (61) étant disposé au niveau de la sortie de flux sanguin (90) et configuré pour mesurer un débit de sang qui est renvoyé au patient (P).

14. Système de pompe à sang-oxygénateur selon l'une quelconque des revendications précédentes, dans lequel la première pompe à sang (11) et le premier oxygénateur (20) sont configurés comme une première unité intégrée (251), et la seconde pompe à sang (13) et le second oxygénateur (21) sont configurés comme une seconde unité intégrée (252).

15. Agencement de transport (500 ; 900), configuré pour transporter le système de pompe à sang-oxygénateur selon l'une quelconque des revendications précédentes qui comprend en outre un port d'alimentation électrique pour fournir de l'énergie à l'unité de commande de pompe, l'agencement de transport comprenant en outre un mécanisme de verrouillage intégré qui est configuré pour se connecter à l'entrée de flux sanguin (80), à la sortie de flux sanguin (90), à une entrée d'oxygène, à une sortie d'oxygène et au port d'alimentation électrique du système de pompe à sang-oxygénateur en une seule action d'amarrage.
